# FASCICULE DE BREVET EUROPEEN

(11) **EP 1 356 207 B1**
(45) Date de publication et mention de la délivrance du brevet: **20.09.2006**
(21) Numéro de dépôt: 02737615.1
(22) Date de dépôt: 24.01.2002
(51) Int. Cl.: F04B 43/12, F04B 43/00, A61M 5/168

(54) **DETECTEUR D'OBSTRUCTION POUR POMPE PERISTALTIQUE ROTATIVE**
VERSTOPFUNGSDETEKTOR FÜR DREHSCHLAUCHPUMPE
OCCLUSION DETECTOR FOR ROTARY PERISTALTIC PUMP

(30) Priorité: 31.01.2001 EP 01810096
(43) Date de publication de la demande: 29.10.2003
(73) Titulaire: Precimedix S.A., 2000 Neuchâtel (CH)
(72) Inventeur: RAY, Claude, CH-2205 Montezillon (CH)
(74) Mandataire: GLN
(86) Numéro de dépôt international: PCT/CH2002/000038
(87) Numéro de publication internationale: WO 2002/061281

(56) Documents cités:
- EP-A- 0 745 400
- US-A- 5 215 450
- US-A- 5 791 881
- US-A- 6 149 394

## Description

La présente invention se rapporte aux pompes péristaltiques rotatives. Elle concerne, plus particulièrement, un détecteur du mauvais fonctionnement, notamment dû à une obstruction, d'une pompe péristaltique rotative miniaturisée notamment destinée à l'injection de solutions de médicaments.

Les pompes miniaturisées à usage médical sont connues depuis plusieurs années. Légères et de faibles dimensions, elles sont portées par le patient de façon discrète et sans inconfort et permettent de lui administrer, par voie sous-cutanée ou intraveineuse, en continu ou selon un programme déterminé, des quantités contrôlées de solutions médicamenteuses, sans qu'il soit, pour autant, alité et relié à un appareil encombrant, coûteux et bruyant.

De telles pompes sont, souvent, de type péristaltique rotatif. Leur principe consiste à disposer d'un tuyau en matière plastique déformable relié à un réservoir contenant la solution de médicament et à l'écraser localement contre une pièce d'appui de forme arrondie au moyen de galets presseurs montés sur un rotor entraîné par un moteur agissant par l'intermédiaire d'un train d'engrenages. Le liquide est ainsi aspiré du réservoir et poussé vers le sortie pour être injecté dans le corps du patient.

Le brevet FR 2 753 235, par exemple, décrit une pompe de ce type.

Lors de la conception de telles pompes, il est particulièrement important de se préoccuper du problème que peut poser l'obstruction du tuyau soit parce qu'il est accidentellement pincé soit parce que le corps du patient s'oppose à l'injection du médicament. Il est tout aussi important de s'assurer que le rotor lui-même n'est pas arrêté et effectue correctement sa rotation. Dans les deux cas il est, alors, nécessaire de déclencher une alarme informant le patient que l'injection ne se fait plus normalement afin qu'il alerte immédiatement les personnes responsables.

Le but de la présente invention est de fournir un détecteur du fonctionnement défectueux de la pompe, notamment pour cause d'obstruction de son tuyau, qui soit à la fois fiable et de faible prix de revient.

De façon plus précise, l'invention concerne un détecteur du mauvais fonctionnement d'une pompe péristaltique dans laquelle un tuyau flexible contenant un fluide est écrasé localement contre une pièce d'appui au moyen de galets montés sur un rotor entraîné par un moteur, ce détecteur étant caractérisé en ce qu'il comporte:
- une bague montée coulissante sur le tuyau entre deux positions extrêmes,
- des moyens d'entraînement, actionnés par le rotor, pour soumettre ladite bague à un mouvement de va et vient déterminé entre lesdites positions, et
- des moyens de détection répondant à un mouvement de va et vient anormal de la bague par la production d'un signal d'alarme.

De préférence, le diamètre intérieur de la bague est tel qu'elle coulisse librement sur le tuyau flexible tant que le fluide qu'il véhicule s'écoule normalement mais que son déplacement soit stoppé lorsque, en raison d'une obstruction, le fluide ne s'écoule plus normalement et provoque un gonflement du tuyau. Il est alors avantageux que, dans sa portion parcourue par la bague, le tuyau présente, localement, une paroi amincie de manière à ce qu'en cas d'obstruction, son gonflement se fasse à cet endroit.

Selon un mode de réalisation préféré, les moyens d'entraînement comportent:
- au moins un plot solidaire du rotor,
- un organe d'entraînement actionné par ledit plot sur une partie de sa course et agissant à son tour sur la bague pour la déplacer, dans une direction, de sa première à sa deuxième position extrême, et
- un ressort agissant sur la bague pour la déplacer, dans la direction opposée, dès qu'elle n'est plus soumise à l'action de l'organe d'entraînement, de sa deuxième à sa première position extrême.

L'organe d'entraînement susmentionné est, avantageusement, un ressort en V, dont la pointe est fixe, dont une branche est actionnée par ledit plot et dont l'autre branche agit sur la bague.

De préférence, les moyens d'entraînement comportent trois plots disposés a 120° les uns des autres sur le rotor.

Avantageusement, les moyens de détection comportent:
- un premier interrupteur fermé par la bague lorsque celle-ci est dans sa première position et ouvert dès qu'elle n'est plus dans cette position,
- un deuxième interrupteur fermé par la bague lorsque celle-ci est dans sa deuxième position et ouvert dès qu'elle n'est plus dans cette position, et
- un circuit ayant en mémoire des informations représentatives d'instants auxquels doivent intervenir la fermeture et l'ouverture de ces interrupteurs lorsque le mouvement de va et vient de la bague s'effectue selon un rythme déterminé correspondant à un bon fonctionnement de la pompe, et ayant pour fonction de:
   . produire des informations représentatives des instants auxquels s'effectuent la fermeture et l'ouverture des interrupteurs,
   . comparer les informations en mémoire avec les informations provenant des interrupteurs, et
   . produire ledit signal d'alarme lorsque l'écart entre les instants effectifs d'ouverture et de fermeture des interrupteurs et les instants d'ouverture et de fermeture en mémoire dépassent une valeur déterminée correspondant à un fonctionnement anormal de la pompe.

Selon une réalisation préférée, lesdits instants sont ceux auxquels la bague successivement:
- quitte sa première position,
- atteint sa deuxième position,
- quitte cette deuxième position, et
- retrouve la première position.

De préférence, lesdites informations sont le nombre d'impulsions fournies au moteur, à partir du moment où la bague quitte sa première position, pour que celle-ci atteigne sa deuxième position, quitte cette position puis retrouve la première position.

La bague est, avantageusement, réalisée en métal et lesdits interrupteurs comportent deux bornes fixes contre lesquelles elle est appliquée lorsqu'elle occupe respectivement ses deux positions extrêmes, et une borne mobile solidaire de la bague.

D'autres caractéristiques de l'invention ressortiront de la description qui va suivre, faite en regard du dessin annexé dans lequel:
- la figure 1 est une vue d'ensemble d'une pompe péristaltique équipée d'un détecteur selon l'invention;
- la figure 2 est une vue agrandie de l'endroit de la pompe où se trouve la partie mécanique détecteur;
- la figure 3 est une vue en coupe selon A-A du détecteur;
- la figure 4 est un schéma de principe montrant la liaison de la partie mécanique du détecteur à sa partie électronique;
- la figure 5 sert à expliquer le fonctionnement du détecteur; enfin,
- la figure 6 présente la séquence des opérations effectuées dans la partie électronique du détecteur.

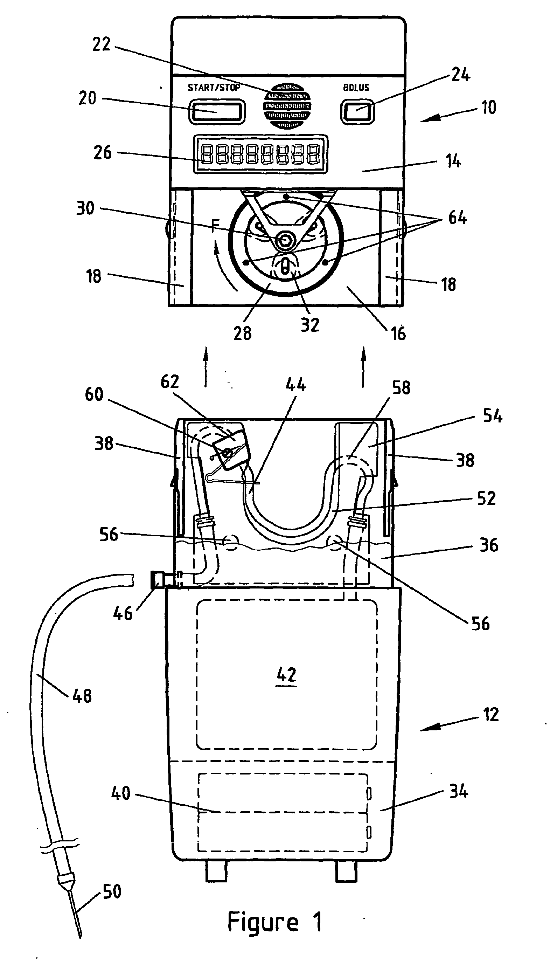

On se référera, tout d'abord, à la figure 1 qui montre une pompe péristaltique à cassette pour l'injection de médicaments liquides. Cette pompe est formée d'un module pompe 10 et d'une cassette 12 qui s'accouplent de manière amovible.

La pompe proprement dite est décrite, de manière très détaillée, notamment dans le brevet FR 2 753 235 déjà cité. La présente description se limitera donc à ses éléments essentiels.

En bref, donc, le module pompe 10 comporte un boîtier en plastique rigide 14 dont la partie côté cassette possède seulement un fond 16 et deux coulisses latérales parallèles 18 servant à la mise en place de la cassette 12 à la manière d'un tiroir.

Sur sa face supérieure, le boîtier 14 comporte un bouton START/STOP 20 servant à commander le démarrage et l'arrêt de la pompe, un avertisseur d'alarme acoustique 22, un bouton BOLUS 24 servant à déclencher l'administration de doses additionnelles de médicament et un affichage LCD 26.

Le boîtier laisse apparaître, entre ses deux coulisses 18, un rotor 28 monté en libre rotation autour d'un axe 30 fixé sur le fond 16 du boîtier et entraîné en rotation au moyen d'un moteur par l'intermédiaire d'un train d'engrenages (non visibles sur la figure).

Le rotor 28 porte trois galets cylindriques 32 disposés à 120° l'un de l'autre et montés libres en rotation autour d'axes parallèles à l'axe 30.

La cassette 12 comporte un boîtier en plastique rigide 34 dont la partie côté module pompe possède seulement une face supérieure 36 et deux bras latéraux parallèles 38 destinés à s'insérer dans les coulisses 18 du module pompe 10. Le reste de la cassette 12 est occupé par deux piles 40 et une poche en plastique 42 remplie d'un médicament liquide, toutes disposées sous des couvercles.

La liaison électrique entre la cassette 12 et le module pompe 10 est assurée par des pistes conductrices (non représentées) déposées sur leurs boîtiers respectifs.

La poche 42 est reliée à un tuyau en plastique 44 qui prend place entre les deux bras 38 et dont l'extrémité débouche à l'extérieur de la cassette où elle est obturée par un bouchon 46. C'est au moment de la mise en service de la pompe que ce bouchon sera enlevé pour permettre de brancher un tuyau flexible 48 se terminant par une aiguille d'injection 50.

Dans sa portion comprise entre les bras 38, le tuyau 44 est appliqué contre un pièce d'appui arrondie 52 en forme de U dont le rayon est légèrement supérieur à celui du cercle que parcourt la face externe des galets 32. La pièce 52 fait partie d'une plaque en plastique rigide 54 fixée sous la face supérieure 36 par enclipsage sur deux clavettes 56. C'est sur cette pièce en U 52 que, lors de la rotation du rotor 28 dans le sens de la flèche F, ses trois galets 32 viennent tour à tour écraser le tuyau 44 et pousser ainsi vers l'extérieur, par un mouvement péristaltique, le liquide contenu dans la poche 42.

De chaque côté de la pièce d'appui 52, la plaque 54 est percée d'un canal 58 dans lequel prend place le tuyau 44.

On remarquera, fixé à la plaque 54 par une vis 60 et monté sur le tuyau 44 en aval de la pièce d'appui 52, un élément 62 qui a pour fonction de détecter toute obstruction à l'injection du médicament liquide dans le corps du patient et d'y réagir par l'enclenchement de l'alarme acoustique 22 et l'inscription d'un message sur l'affichage LCD 26.

Il est important de noter que la portion du tuyau 44 qui est soumise à l'action des galets 32 et travers l'élément 62 a un diamètre extérieur réduit qui lui confère davantage de souplesse afin, non seulement, de faciliter sa compression par les galets, mais également, de permettre son gonflement en cas d'obstruction en aval.

On remarquera aussi que le rotor 28 porte, sur sa face supérieure, trois plots métalliques 64 disposés à 120° les uns des autres et servant, comme cela va être maintenant décrit en détail, à l'actionnement de l'élément de détection 62.

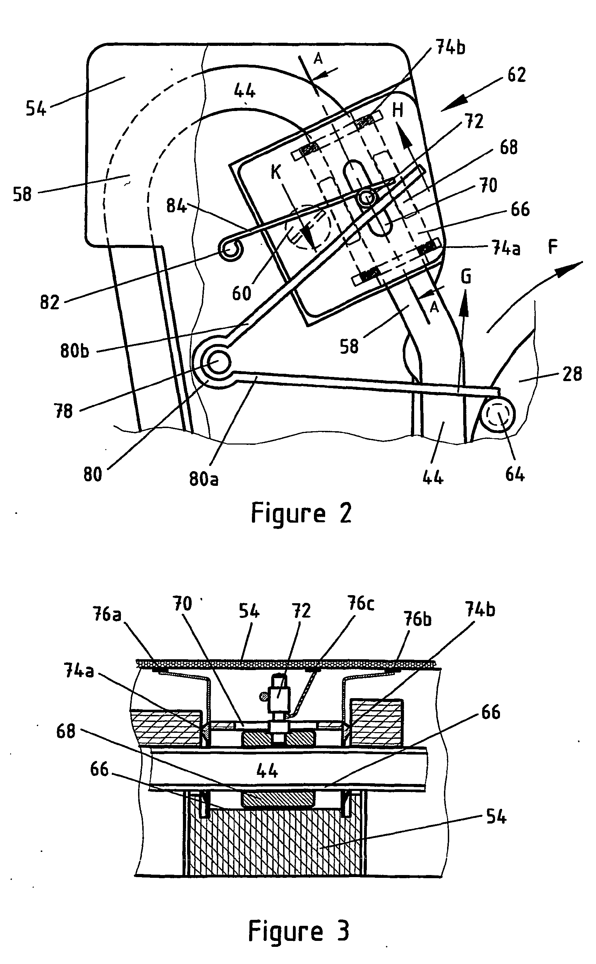

Sur les figures 2 et 3, on voit que l'élément 62, réalisé en plastique rigide, est disposé à l'intérieur d'un logement pratiqué dans la face supérieure de la plaque 54. Le fond de ce logement comporte une rainure semi-cylindrique qui délimite, avec une rainure identique formée à la base de l'élément 62, un canal cylindrique 66 débouchant, à ses deux extrémités, dans le canal 58 servant de guide au tuyau 44.

Le canal 66 reçoit une bague 68, avantageusement réalisée en laiton, qui enserre le tuyau 44 dans sa portion plus souple. Le diamètre intérieur de cette bague est ajusté de manière à ce qu'en l'absence de toute obstruction du tuyau en aval, qui aurait pour effet de la faire gonfler, elle puisse coulisser librement sur lui, mais qu'en cas de gonflement dû à une obstruction son mouvement soit empêché. Quant au diamètre extérieur de la bague 68, il est légèrement inférieur au diamètre du canal 66 de manière à ce que celui-ci ne gène en rien son déplacement.

L'élément 62 est percé, à sa partie supérieure, d'une rainure longitudinale 70 parallèle à l'axe de son canal 66 et débouchant sur lui. Par ailleurs, la bague 68 est solidaire d'un arbre 72, réalisé en laiton ou en acier, qui est perpendiculaire à son axe et traverse la rainure 70. Celle-ci limite ainsi la course de la bague 68.

Les deux extrémités du canal 66 sont munies chacune d'une lame de contact métallique en forme de U 74A et 74B laissant passer le tuyau et contre laquelle la bague 68 vient s'appliquer lorsqu'elle se trouve dans l'une ou l'autre de ses positions de fin de course. Chacune de ces lames constitue une borne fixe qui est respectivement reliée à une piste conductrice 76A et 76B disposée sur la face inférieure de la plaque 54.

Cette dernière porte une goupille 78 servant de point de maintien et de pivotement à une lame ressort métallique en V 80 dont l'un des bras 80a est, lors de la progression du rotor 28 selon F, poussé à son extrémité selon G successivement par les trois plots 64. L'extrémité de l'autre bras 80b du ressort est, elle, en appui contre l'arbre 72 de la bague 68 qu'elle pousse ainsi selon H, parallèlement à son axe, sous l'effet des plots 64.

La face inférieure de la plaque 54 porte une deuxième goupille 82 servant de point d'attache à une lame ressort métallique 84 dont l'extrémité est en appui contre l'arbre 72 de manière à exercer sur lui une poussée, selon K, dans le sens opposé à celle qu'exerce le ressort en V 80. La lame 84 constitue une borne mobile qui est reliée à une troisième piste conductrice 76C disposée sur la face inférieure de la plaque.

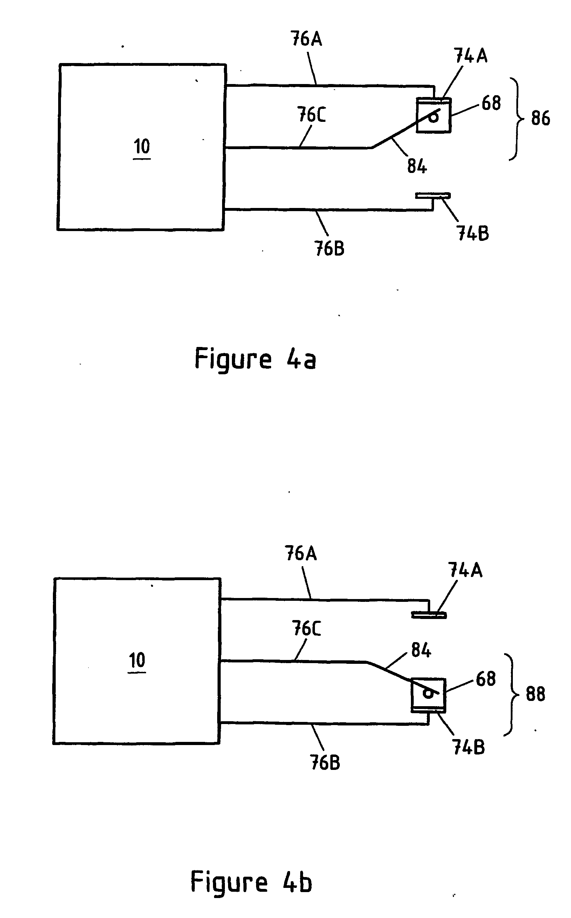

Comme le montre la figure 4, les trois pistes 76A, 76B et 76C aboutissent au module pompe 10 dont le circuit de commande a pour rôle d'observer la séquence des instants auxquels:
- selon la figure 4a, la bague 68 est en appui contre la borne fixe 74A (première position), ce qui la relie électriquement à la borne mobile 84 et connecte ainsi entre elles les pistes 76A et 76C;
- selon la figure 4b, la bague 68 est en appui contre la borne fixe 74B (deuxième position), ce qui la relie électriquement à la borne mobile 84 et connecte ainsi entre elles les pistes 76B et 76C.

Sur la figure 4, on a schématisé ces deux situations en montrant qu'elles correspondent à la fermeture ou l'ouverture de deux interrupteurs 86 et 88.

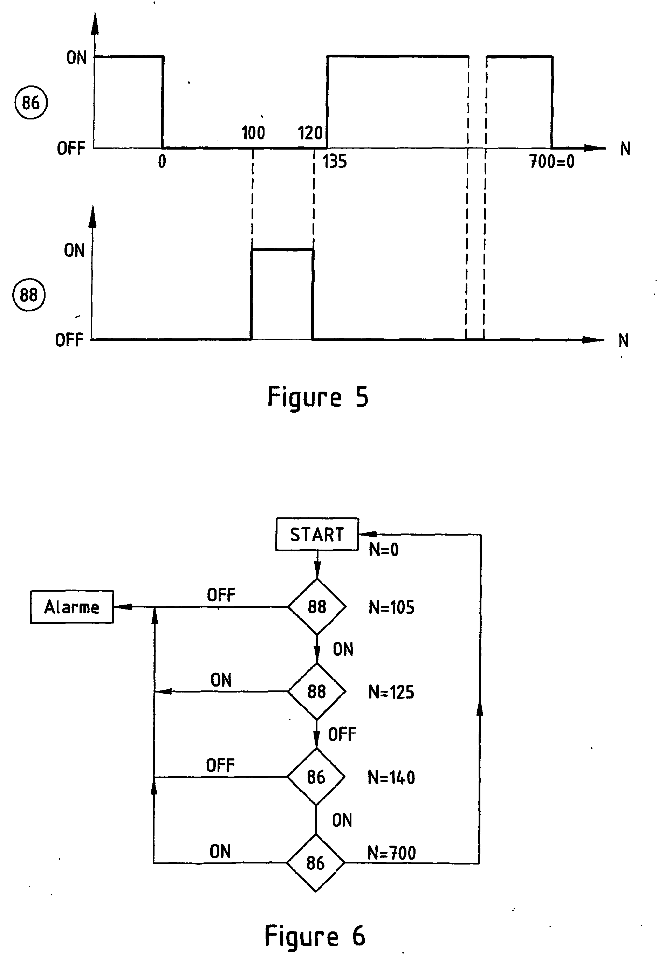

Le fonctionnement du détecteur selon l'invention va être maintenant décrit avec l'aide des figures 5 et 6, en prenant comme exemple un système dans lequel un tour complet du rotor 28 correspond à 2100 impulsions appliquées à son moteur. Cela signifie que l'un des trois plots 64 vient au contact du ressort en V 80 toutes les 700 impulsions motrices.

Le circuit de la pompe a en mémoire des informations représentatives d'instants auxquels doivent intervenir la fermeture et l'ouverture des interrupteurs 86 et 88 lorsque le mouvement de va et vient de la bague s'effectue selon un rythme déterminé correspondant à un bon fonctionnement de la pompe. Ce circuit est, de préférence, un microprocesseur qui a pour fonction de:
. produire des informations représentatives des instants auxquels s'effectuent la fermeture et l'ouverture des deux interrupteurs,
. comparer les informations en mémoire avec les informations provenant des interrupteurs, et
. produire ledit signal d'alarme lorsque l'écart entre les instants effectifs d'ouverture et de fermeture des interrupteurs et les instants d'ouverture et de fermeture en mémoire dépassent une valeur déterminée correspondant à un fonctionnement anormal de la pompe.

Selon un mode de réalisation préféré, le circuit s'intéresse aux instants auxquels la bague 68 successivement:
- quitte sa première position et ouvre donc l'interrupteur 86,
- atteint sa deuxième position et ferme donc l'interrupteur 88,
- quitte cette deuxième position et ouvre donc l'interrupteur 88, puis
- retrouve la première position et ferme donc l'interrupteur 86.

Dans l'exemple décrit, les informations en mémoire sont le nombre N d'impulsions fournies au moteur, à partir du moment où la bague quitte sa première position, pour que celle-ci:
- atteigne sa deuxième position, soit pour N = 100;
- quitte cette position, soit pour N = 120,
- retrouve la première position, soit pour N = 135,
- quitte cette position, soit pour N = 700.

Le circuit est donc équipé d'un compteur des impulsions fournies au moteur.

On part d'un état où aucun des plots 64 n'agit sur le ressort en V 80. Sous l'action de la lame ressort 84, la bague 68 se trouve ainsi en appui contre la lame 74A. L'interrupteur 86 est donc fermé (ON) et l'interrupteur 88 ouvert (OFF). Le compteur est alors à 0.

Comme le montre la figure 6, le cycle débute au moment où l'un des plots 64 met, par l'intermédiaire du ressort 80, la bague en mouvement selon H. En quittant la lame 74A, elle provoque donc l'ouverture de l'interrupteur 86, l'interrupteur 88 restant ouvert. Le circuit se met alors à compter les impulsions motrices.

A la 100^{ème} impulsion, si tout est normal, la bague arrive contre la lame 74B et provoque ainsi la fermeture de l'interrupteur 88. En revanche, dans le cas où une obstruction du tuyau 44 provoque son gonflement, la bague se trouve empêchée de progresser normalement et l'interrupteur 88 ne se ferme pas. Si ce dernier est toujours ouvert (OFF) à la 105^{ème} impulsion (N = 105), c'est qu'il y a effectivement un blocage de la bague dû à une sérieuse obstruction du tuyau. Le circuit réagit alors en déclenchant l'activation de l'a11larme acoustique 22 et l'inscription d'un message d'alerte sur l'afficheur 26. Le porteur de la pompe doit alors, selon les instructions qui lui ont été fournies, la stopper en appuyant sur le bouton 20 et contacter le personnel compétent.

Si aucune anomalie n'a été détectée, la 120^{ème} impulsion correspond à l'instant auquel l'extrémité du ressort en V 80 échappe au plot 64, ce qui provoque la mise en mouvement rapide de la bague selon K sous l'action de la lame ressort 84. Normalement, l'interrupteur 88 s'ouvre donc. Par contre, si un gonflement du tuyau provoque l'immobilisation de la bague, interdisant donc l'ouverture de l'interrupteur 88, et si celui-ci est toujours fermé (ON) à la 125^{ème} impulsion (N = 125), le circuit déclenche l'alarme.

Si rien d'anormal ne s'est passé, c'est à la 135^{ème} impulsion que la bague doit revenir sur la lame 74A et provoquer la fermeture de l'interrupteur 86. Mais si, à la 140^{ème} impulsion (N = 140), cet interrupteur est toujours ouvert (OFF), cela traduit une obstruction du tuyau et entraîne le déclenchement de l'alarme.

Dans le cas d'un fonctionnement normal, la bague va alors, sous l'action de la lame ressort 84, rester appliquée sur la lame 74A, l'interrupteur 86 demeurant donc fermé (ON), jusqu'à ce que le plot 64 suivant remette la bague en mouvement selon H et provoque donc la réouverture de l'interrupteur 86. Cela se produit normalement avec la 700^{ème} impulsion (N = 700). Mais si, pour cause d'obstruction, l'interrupteur 86 reste fermé (ON), il y a déclenchement de l'alarme. Par contre, si tout va bien, un nouveau cycle démarre alors, identique à celui qui vient d'être décrit, après remise à 0 du compteur.

Ainsi est réalisé un détecteur, à la fois simple, fiable et peu coûteux, qui, en s'assurant que la bague effectue normalement son mouvement de va et vient, permet de signaler toute obstruction du tuyau. Bien entendu, même s'il n'y a pas d'obstruction s'opposant au déplacement de la bague, tout mauvais fonctionnement de la pompe (imputable à la pile, au moteur, au train d'engrenages ou au rotor) entraînant un mouvement anormal de la bague sera aussi détecté et signalé.

L'invention permet donc de détecter, non seulement, une obstruction du tuyau, mais aussi, un mauvais fonctionnement de la pompe elle-même.

## Revendications

1. Détecteur du mauvais fonctionnement d'une pompe péristaltique dans laquelle un tuyau flexible (44) contenant un fluide est écrasé localement contre une pièce d'appui (52) au moyen de galets (32) montés sur un rotor (28) entraîné par un moteur, **caractérisé en ce qu'**il comporte:
- une bague (68) montée coulissante sur le tuyau entre deux positions extrêmes,
- des moyens d'entraînement (64, 80, 84), actionnés par le rotor, pour soumettre ladite bague à un mouvement de va et vient déterminé entre lesdites positions, et
- des moyens de détection (86, 88) répondant à un mouvement de va et vient anormal de la bague par la production d'un signal d'alarme.

2. Détecteur selon la revendication 1, **caractérisé en ce que** le diamètre intérieur de la bague (68) est tel qu'elle coulisse librement sur le tuyau flexible (44) tant que le fluide qu'il véhicule s'écoule normalement mais que son déplacement est stoppé lorsque, en raison d'une obstruction, le fluide ne s'écoule plus normalement et provoque un gonflement du tuyau (44).

3. Détecteur selon la revendication 2, **caractérisé en ce que**, dans sa portion parcourue par la bague (68), le tuyau (44) présente, localement, une paroi amincie de manière à ce qu'en cas d'obstruction, son gonflement se fasse à cet endroit.

4. Détecteur selon la revendication 1, **caractérisé en ce que** lesdits moyens d'entraînement comportent:
- au moins un plot (64) solidaire du rotor (28),
- un organe d'entraînement (80) actionné par ledit plot sur une partie de sa course et agissant à son tour sur la bague (68) pour la déplacer, dans une direction, de sa première à sa deuxième position extrême, et
- un ressort (84) agissant sur la bague pour la déplacer, dans la direction opposée, dès qu'elle n'est plus soumise à l'action de l'organe d'entraînement, de sa deuxième à sa première position extrême.

5. Détecteur selon la revendication 4, **caractérisé en ce que** ledit organe d'entraînement (80) est un ressort en V, dont la pointe est fixe, dont une branche (80a) est actionnée par ledit plot (64) et dont l'autre branche (80b) agit sur la bague (68).

6. Détecteur selon l'une des revendications 4 et 5, **caractérisé en ce que** les moyens d'entraînement comportent trois plots (64) disposés a 120° les uns des autres sur le rotor.

7. Détecteur selon la revendication 1, **caractérisé en ce que** lesdits moyens de détection comportent:
- un premier interrupteur (86) fermé par la bague (68) lorsque celle-ci est dans sa première position et ouvert dès qu'elle n'est plus dans cette position,
- un deuxième interrupteur (88) fermé par la bague (68) lorsque celle-ci est dans sa deuxième position et ouvert dès qu'elle n'est plus dans cette position, et
- un circuit ayant en mémoire des informations représentatives d'instants auxquels doivent intervenir la fermeture et l'ouverture de ces interrupteurs lorsque le mouvement de va et vient de la bague s'effectue selon un rythme déterminé correspondant à un bon fonctionnement de la pompe, et ayant pour fonction de:
. produire des informations représentatives des instants auxquels s'effectuent la fermeture et l'ouverture des interrupteurs,
. comparer les informations en mémoire avec les informations provenant des interrupteurs, et
. produire ledit signal d'alarme lorsque l'écart entre les instants effectifs d'ouverture et de fermeture des interrupteurs et les instants d'ouverture et de fermeture en mémoire dépassent une valeur déterminée correspondant à un fonctionnement anormal de la pompe.

8. Détecteur selon la revendication 7, **caractérisé en ce que** lesdits instants sont ceux auxquels la bague successivement:
- quitte sa première position,
- atteint sa deuxième position,
- quitte cette deuxième position, et
- retrouve la première position.

9. Détecteur selon l'une des revendications 7 et 8, **caractérisé en ce que** lesdites informations sont le nombre d'impulsions fournies au moteur, à partir du moment où la bague quitte sa première position, pour que celle-ci atteigne sa deuxième position, quitte cette position puis retrouve la première position.

10. Détecteur selon l'une des revendication 7 à 9, **caractérisé en ce que** la bague (68) est réalisée en métal et **en ce que** lesdits interrupteurs (86, 88) comportent deux bornes fixes (74A, 74B) contre lesquelles elle est appliquée lorsqu'elle occupe respectivement ses deux positions extrêmes, et une borne mobile (84) solidaire de la bague.

## Claims

1. Detector for detecting malfunction in a peristaltic pump wherein a flexible tubing (44) containing a fluid is locally compressed against a support part (52) by means of rollers (32) mounted on a rotor (28) driven by a motor, **characterized in that** it includes:
a ring (68) mounted so as to slide on the tubing between two extreme positions,
drive means (64, 80, 84), activated by the rotor, for subjecting said ring to a specific reciprocating motion between said positions, and
detection means (86, 88) reacting to an abnormal reciprocating motion of the ring by producing an alarm signal.

2. Detector according to claim 1, **characterized in that** the internal diameter of the ring (68) is such that it slides freely on the flexible tubing (44) as long as the fluid flows normally, but that its movement is stopped when, because of an occlusion, the fluid no longer flows normally and causes the tubing (44) to swell.

3. Detector according to claim 2, **characterized in that**, in the portion in which the ring (68) travels, the tubing (44) has, locally, a thinned wall so that, in the event of occlusion, the swelling occurs at that location.

4. Detector according to claim 1, **characterized in that** said drive means include:.
- at least one stud (64) attached to the rotor (28);
- a drive member (80) activated by said stud on a part of its travel and acting in turn on the ring to move it, in one direction, from its first to its second extreme position, and
- a spring (84) acting on the ring to move it, in the opposite direction, as soon as it is no longer subjected to the action of the drive member, from its second to its first extreme position.

5. Detector according to claim 4, **characterized in that** said drive member (80) is a V-shaped spring, whose tip is fixed, and one of whose branches (80a) is activated by said stud (64) and the other branch (80b) acts on the ring (68).

6. Detector according to claims 4 or 5, **characterized in that** the drive means include three studs (64) arranged at 120° from each other on the rotor.

7. Detector according to claim 1, **characterized in that** said detection means include:
- a first switch (86) closed by the ring (68) when the latter is in its first position and open as soon as it is no longer **in that** position,
- a second switch (88) closed by the ring (68) when the latter is in its second position and open as soon as it is no longer **in that** position, and
- a circuit that has, in its memory, data representative of the moments at which said switches have to close and open when the reciprocating motion of the ring occurs according to a determined rhythm corresponding to proper operation of the pump, and whose function is to:
• produce data representative of the moments at which closing and opening of the switches occurs,
• compare the data contained in the memory with the data from the switches, and
• produce said alarm signal when the difference between the actual switch opening and closing moments and the opening and closing moments contained in the memory exceed a determined value corresponding to abnormal operation of the pump.

8. Detector according to claim 7, **characterized in that** said moments are those at which the ring successively:
- leaves its first position,
- reaches its second position,
- leaves this second position, and
- returns to the first position.

9. Detector according to claims 7 or 8, **characterized in that** said data is the number of pulses provided to the motor, from the moment when the ring leaves its first position, so that the latter reaches its second position, leaves that position, then returns to the first position.

10. Detector according to any of claims 7 to 9, **characterized in that** the ring (68) is made of metal and **in that** said switches (86, 88) include two fixed terminals (74A, 74B) against which the ring is applied when it occupies respectively its two extreme positions, and a mobile terminal (84) attached to the ring.

## Patentansprüche

1. Detektor für die Fehlfunktion einer Peristaltikpumpe, in der ein biegsames Rohr (44), das ein Fluid enthält, lokal gegen einen Stützteil (52) mit Hilfe von Rollen (32), die auf einem von einem Motor angetriebenen Rotor (28) montiert sind, gedrückt wird, **dadurch gekennzeichnet, dass** er umfasst:
- einen gleitend auf dem Rohr zwischen zwei äußersten Positionen montierten Ring (68),
- Antriebsmittel (64, 80, 84), die vom Rotor betätigt werden, um den Ring einer bestimmten Hin- und Herbewegung zwischen den Positionen zu unterziehen, und
- Erfassungsmittel (86, 88), die auf eine abnormale Hin- und Herbewegung des Ringes mit der Erzeugung eines Alarmsignals reagieren.

2. Detektor nach Anspruch 1, **dadurch gekennzeichnet, dass** der Innendurchmesser des Ringes (68) derart ist, dass er frei auf dem biegsamen Rohr (44) gleitet, so lange das Fluid, das es befördert, normal fließt, dass aber seine Verschiebung angehalten wird, wenn auf Grund einer Verstopfung das Fluid nicht mehr normal fließt und zu einer Ausbuchtung des Rohrs (44) führt.

3. Detektor nach Anspruch 2, **dadurch gekennzeichnet, dass** das Rohr (44) in seinem vom Ring (68) durchlaufenen Teil lokal eine dünnere Wand aufweist, so dass im Falle seiner Verstopfung seine Ausbuchtung an dieser Stelle entsteht.

4. Detektor nach Anspruch 1, **dadurch gekennzeichnet, dass** die Antriebsmittel umfassen:
- mindestens ein mit dem Rotor (28) verbundenes Kontaktstück (64),
- ein Antriebselement (80), das von dem Kontaktstück auf einem Teil seines Weges betätigt wird und seinerseits auf den Ring (68) einwirkt, um ihn in eine Richtung von seiner ersten in seine zweite äußerste Position zu verschieben, und
- eine Feder (84), die auf den Ring einwirkt, um ihn in die entgegengesetzte Richtung von seiner zweiten in seine erste äußerste Position zu verschieben, sobald er nicht mehr der Wirkung des Antriebselements ausgesetzt ist.

5. Detektor nach Anspruch 4, **dadurch gekennzeichnet, dass** das Antriebselement (80) eine V-förmige Feder ist, deren Spitze fest ist, deren einer Abschnitt (80a) von dem Kontaktstück (64) betätigt wird, und deren anderer Abschnitt (80b) auf den Ring (68) einwirkt.

6. Detektor nach einem der Ansprüche 4 und 5, **dadurch gekennzeichnet, dass** die Antriebsmittel drei Kontaktstücke (64) umfassen, die in einem Winkel von 120° zueinander auf dem Rotor angeordnet sind.

7. Detektor nach Anspruch 1, **dadurch gekennzeichnet, dass** die Erfassungsmittel umfassen:
- einen ersten Schalter (86), der vom Ring (68) geschlossen wird, wenn sich dieser in seiner ersten Position befindet, und der geöffnet wird, sobald er sich nicht mehr in dieser Position befindet,
- einen zweiten Schalter (88), der vom Ring (68) geschlossen wird, wenn sich dieser in seiner zweiten Position befindet, und der geöffnet wird, sobald er sich nicht mehr in dieser Position befindet, und
- eine Schaltung, die Informationen zu den Zeitpunkten, zu denen das Schließen und Öffnen dieser Schalter stattfinden soll, gespeichert hat, wenn die Hin- und Herbewegung des Ringes nach einem bestimmten Rhythmus erfolgt, der einer ordnungsgemäßen Funktion der Pumpe entspricht, und die folgende Aufgabe hat:
* Erstellung von Informationen zu den Zeitpunkten, zu denen das Schließen und Öffnen der Schalter erfolgt,
* Vergleich der gespeicherten Informationen mit den von den Schaltern kommenden Informationen, und
* Erzeugung des Alarmsignals, wenn der Abstand zwischen den tatsächlichen Zeitpunkten des Öffnens und Schließens der Schalter und den gespeicherten Zeitpunkten des Öffnens und Schließens einen bestimmten Wert übersteigt, der einer abnormalen Funktion der Pumpe entspricht.

8. Detektor nach Anspruch 7, **dadurch gekennzeichnet, dass** die Zeitpunkte jene sind, zu denen der Ring nach einander:
- seine erste Position verlässt,
- seine zweite Position erreicht,
- diese zweite Position verlässt, und
- wieder in die erste Position zurückkehrt.

9. Detektor nach einem der Ansprüche 7 und 8, **dadurch gekennzeichnet, dass** die Informationen die an den Motor gelieferten Impulszahlen ab dem Zeitpunkt sind, zu dem der Ring seine erste Position verlässt, um seine zweite Position zu erreichen, diese Position verlässt und dann in seine erste Position zurückkehrt.

10. Detektor nach einem der Ansprüche 7 bis 9, **dadurch gekennzeichnet, dass** der Ring (68) aus Metall hergestellt ist, und dass die Schalter (86, 88) zwei feste Klemmen (74A, 74B), gegen die er angelegt wird, wenn er seine jeweils beiden äußersten Positionen einnimmt, und eine bewegliche Klemme (84), die mit dem Ring verbunden ist, umfassen.
